# EUROPÄISCHE PATENTSCHRIFT

(11) **EP 0 415 264 B1**
(45) Veröffentlichungstag und Bekanntmachung des Hinweises auf die Patenterteilung: **29.06.1994**
(21) Anmeldenummer: 90116140.6
(22) Anmeldetag: 23.08.1990
(51) Int. Cl.: C07D 265/30, C07D 207/10, C07D 211/38, C25B 3/08

(54) **Verfahren zur Herstellung perfluorierter heterocyclischer Verbindungen und nach diesem Verfahren hergestellte Verbindungen**
Process for the preparation of perfluorinated heterocyclic compounds and compounds prepared by this process
Procédé pour la préparation de composés hétérocycliques perfluorés et composés préparés selon ce procédé

(30) Priorität: 30.08.1989 DE 3928692; 15.12.1989 DE 3941515
(43) Veröffentlichungstag der Anmeldung: 06.03.1991
(73) Patentinhaber: Kali-Chemie Aktiengesellschaft, D-30173 Hannover (DE)
(72) Erfinder: Meinert, Hasso, D-7900 Ulm (DE); Fackler, Rudolf, D-7913 Senden (DE); Mader, Jürgen, D-7900 Ulm (DE); Reuter, Peter, D-7900 Ulm-Lehr (DE)
(74) Vertreter: Lauer, Dieter, Dr.

(56) Entgegenhaltungen:
- DD-A- 275 079
- US-A- 4 534 978
- CHEMICAL ABSTRACTS, Band 109, Nr. 1, 4. Juli 1988, Seite 191, ZusammenfassungNr. 1969d, Columbus, Ohio, US; V.V. OBRAZTSOV et al.: "Study of physicochemical properties of fluorocarbon inducers of cytochrome P 450 in rat liver. Endoplasmic reciculum membranes", & BIOKHIMIYA (MOSCOW) 1988, 53(4), 613-19

## Beschreibung

Die vorliegende Erfindung betrifft ein Verfahren zur Herstellung von perfluorierten heterocyclisch substituierten Cycloalkyl-Verbindungen und deren Gemischen mit entsprechenden n-Alkyl-Verbindungen ausgehend von entsprechenden unfluorierten Cycloalkenylverbindungen.

Die erfindungsgemäß herstellbaren Verbindungen gehören zum Typ der sogenannten Perfluorkohlenstoffe. Als Perfluorkohlenstoffe (=Perfluorcarbone) bekannte, bei Raumtemperatur unter Normaldruck flüssige bis wachsartige perfluorierte organische Verbindungen, im folgenden "Perfluorcarbone" genannt, sind wasserunlösliche chemisch und biologisch inerte Verbindungen, welche aus Kohlenstoff und Fluor bestehen und gegebenenfalls noch Heteroatome, zum Beispiel Stickstoff oder Sauerstoff, enthalten. Derartige Verbindungen sind bekannt, z.B. auf den europäischen Patentanmeldungen, Veröffentlichungsnummer 77 114, 99 652 und 151 697. Die Perfluorcarbonmoleküle sind durch eine einheitliche Schale von Fluoratomen hervorragend abgeschirmt. Daher sind Perfluorcarbone chemisch und physiologisch außerordentlich inert, also ungiftig. Wegen ihrer extrem geringen intermolekularen Kräfte haben Perfluorcarbone einen im Verhältnis zu ihren Molekülmassen tiefen Siedepunkt und eine außerordentlich geringe Oberflächenspannung. Aus den sehr schwachen intermolekularen Kräften resultiert auch die Fähigkeit der Perfluorcarbone große Mengen von Gasen, beispielsweise Sauerstoff und Kohlendioxid zu lösen. Aufgrund dieser Eigenschaften, insbesondere der Fähigkeit, Sauerstoff physikalisch zu lösen und zu transportieren, haben Perfluorcarbone in der Medizin Anwendung gefunden zur Herstellung von wäßrigen Sauerstoff transportierenden Perfluorcarbonemulsionen, welche zum Beispiel als Blutersatzmittel oder Perfusionsmedien eingesetzt werden. Aus einer in Chemical Abstracts, Abstract No 109; 1969d, C.A. Vol. 109, 1988 referierten russischen Untersuchung über Cytochrom P 450 Induktion in Rattenlebermicrosomen durch Perfluorcarbone ist das Perfluoro-N-cyclohexylmorpholin bekannt. Ferner sind Perfluorcarbone auch zur Anwendung in anderen technischen Gebieten, in welchen ungiftige und chemisch inerte flüssige oder wachsartige Substanzen benötigt werden, bzw. inerte Substanzen mit einem Lösevermögen für Gase benötigt werden, geeignet.

Bisher bekannte Verfahren zur Herstellung von perfluorierten heterocyclisch substituierten Cycloalkylverbindungen gehen von entsprechenden gesättigten unfluorierten Cycloylkyl-Verbindungen aus, welche ihrerseits in aufwendigen Herstellungsverfahren gewonnen werden müssen. Gegenüber derartigen Verfahren hat das erfindungsgemäße Verfahren den Vorteil, daß es über wenige Reaktionsstufen leicht zugängige Ausgangsstoffe verwendet.

Aufgabe der vorliegenden Erfindung ist es, ein wirtschaftliches und verbessertes Verfahren zur Herstellung von perfluorierten heterocyclisch substituierten Cycloalkylverbindungen zu entwickeln.

Es wurde nun gefunden, daß perfluorierte heterocyclisch substituierte Cycloalkyl-Verbindungen und entsprechende n-Hexyl-Verbindungen durch elektrochemische Fluorierung von entsprechenden unfluorierten ungesättigten Cycloalkylen-Verbindungen in guter Ausbeute erhalten werden können.

Gegenstand der vorliegenden Erfindung ist ein Verfahren zur Herstellung von Verbindungen der allgemeinen Formeln Ia und Ib 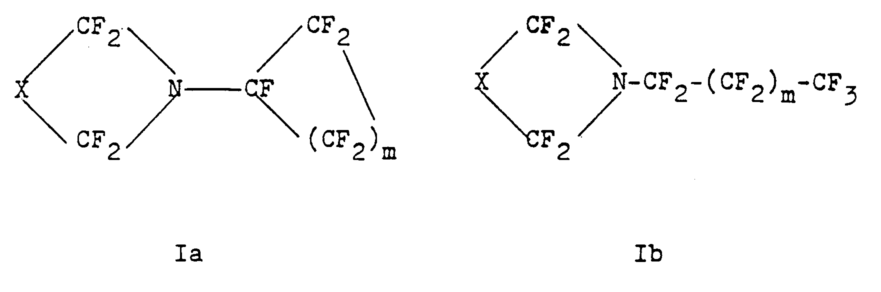
worin m 3 oder 4 bedeutet und X eine -CF₂-O-CF₂-Gruppe, eine -CF₂ -CF₂ -CF₂ -Gruppe oder eine -CF(CF₃ )-CF₂ -Gruppe darstellt,
oder deren Gemischen, dadurch gekennzeichnet, daß man
a) eine Lösung von Verbindungen der allgemeinen Formel II 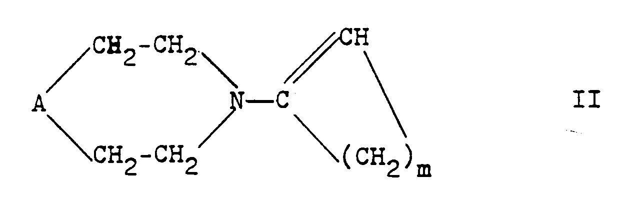 worin m obige Bedeutung besitzt und A Sauerstoff oder eine -CH₂-Gruppe bedeutet, in flüssigem Fluorwasserstoff elektrolysiert und ein die perfluorierten Verbindungen der Formel Ia und Ib neben nur partiell fluorierten Nebenprodukten enthaltendes rohes Reaktionsprodukt abtrennt,
b) das rohe Reaktionsprodukt mit einer Alkali- oder Erdalkalimetallbase, insbesondere einem Alkali- oder Erdalkalimetallhydroxid, in Gegenwart von Wasser und gegebenenfalls einem niederen aliphatischen primären oder sekundären Amin bei einer zur Zersetzung von nur partiell fluorierten Nebenprodukten ausreichend hohen Temperatur behandelt,
c) ein Gemisch der Verbindungen der Formeln Ia und Ib aus einem in Verfahrensstufe b) erhaltenem Reaktionsgemisch isoliert, und dieses gewünnschtenfalls in die Verbindungen der Formel Ia und Ib auftrennt, und
d) gewünschtenfalls erhaltene Isomerengemische aus Verbindungen Ia oder Ib, worin X eine -CF₂-CF₂-CF₂-Gruppe bedeutet und m obige Bedeutung besitzt und dazu isomeren Verbindungen der Formeln Ia oder Ib, worin X eine -CF(CF₃)-CF₂-Gruppe bedeutet, in die einzelnen Isomeren auftrennt.

Es ist als überraschend anzusehen, daß Verbindungen der Formel II ohne Spaltung des Moleküls an der Doppelbindung und Bildung von Spaltprodukten und/oder polymeren Abkömmlingen derselben zu den entsprechenden Gemischen aus perfluorierten Verbindungen der Formel Ia und Ib umgesetzt werden können.

Gegenstand der Erfindung sind ferner nach dem erfindungsgemäßen Verfahren erstmals hergestellte Gemische von Perfluoro-N-cyclohexylmorpholin der Formel I'a mit Perfluoro-N-n-hexylmorpholin der Formel I'b 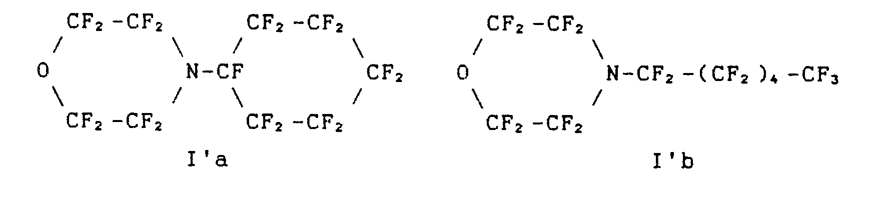
Die erfindungsgemäß herstellbaren perfluorierten heterocyclischen Verbindungen sind aufgrund ihrer chemischen und physiologischen Inertheit und ihrer Fähigkeit Gase, beispielsweise Sauerstoff und Kohlendioxid zu lösen für die vorstehend für Perfluorcarbone angegebenen Verwendungszwecke einsetzbar.

Die Verbindung der Formel I′a zeichnet sich durch ein besonders günstiges Eigenschaftsprofil aus, auf Grund dessen sie und ihre Gemische mit Verbindung I′b sich besonders gut zur Herstellung von wäßrigen zum Sauerstofftransport befähigten Perfluorcarbonemulsionen eignen, welche in der Medizin, z.B. als Blutersatzmittel oder auch zur Organperfusion und -lagerung in der Transplantationschirugie, eingesetzt werden können.

Perfluorcarbone sind hydrophobe mit Wasser nicht mischbare Substanzen. Sie können daher nicht als solche, sondern nur in Form von physiologisch verträglichen wäßrigen Emulsionen in den Blutkreislauf eingeführt werden. Derartige Emulsionen werden bekannterweise mit Hilfe eines physiologisch verträglichen Emulgators hergestellt und stellen Emulsionen vom Öl-in-Wasser-Typ dar.

Neben einer guten physiologischen Verträglichkeit, z.B. dem normalen Blut ähnlichen osmotischen und onkotischen (kolloidosmotischen) Drucken, Fließeigenschaften und pH-Konstanz, und einer guten Sauerstofflösekapazität ist es für als Blutersatzmittel verwendbare Emulsionen wichtig, daß sie eine geeignete Verweildauer im Blutkreislauf aufweisen und anschließend möglichst vollständig und ohne übermäßige Retention in Organen wieder ausgeschieden werden. Als günstig werden Verweilhalbwertzeiten im Körper von ca. einer Woche bis zu einem Monat, insbesondere von ca. 2 - 4 Wochen, angesehen. Ferner ist es wichtig, daß die Emulsionen eine ausreichende Stabilität gegen ein Wachstum der ölphasenpartikel aufweisen.

Perfluorcarbone werden dank ihrer chemischen Inertheit im Körper praktisch nicht metabolisiert und werden in unveränderter Form mit der Atemluft oder durch die Haut ausgeschieden. Die Ausscheidungsrate einzelner Perfluorcarbone ist sehr unterschiedlich und kann von wenigen Tagen bis zu einigen Jahren variieren. Die Ausscheidungsrate der einzelnen Substanzen hängt dabei stark von deren Dampfdruck sowie auch von ihrer Löslichkeit in niedrigpolaren Medien ab, da die Durchtrittsgeschwindigkeit der Perfluorcarbone durch die alveolaren Membranen von diesen Parametern abhängig ist.

Als Maß für die Lipophilie der Perfluorcarbone kann die kritische Lösungstemperatur in n-Hexan (=CTSH), das ist die Temperatur, bei welcher sich das betreffende Perfluorcarbon in einer gleichen Menge n-Hexan löst, dienen. Die Bestimmung der CTSH eines Perfluorcarbons ist daher eine in-vitro-Testmethode, welche ein gutes Indiz für die Verweildauer des Perfluorcarbons im Körper darstellt.

Für eine befriedigende Exhalationsrate sind eine ausreichende Lipophylie und ein ausreichend hoher Dampfdruck vorteilhaft.

Die Verbindung der Formel Ia′ ist als sauerstoffübertragender Bestandteil von als Blutersatzmittel geeigneten Perfluorcarbonemulsionen besonders geeignet, da sie neben einem hohen Sauerstofflösevermögen sowohl eine gute Emulgierbarkeit wie auch für eine geeignete Verweilhalbwertzeit im Körper mit guter Ausscheidungsrate günstige CTSH und Dampfdruck zeigt.

Erfindungsgemäß werden Mischungen aus Verbindungen der Formel Ia und den Nebenprodukten der Formel Ib durch elektrochemische Fluorierung von ungesättigten Verbindungen der Formel II dargestellt, indem man Lösungen der Verbindungen der Formel II in flüssigem Fluorwasserstoff elektrolysiert. Zweckmäßig werden für das Verfahren Lösungen von 4 - 30, vorzugsweise 5 - 10 Gew-% einer Verbindung der Formel II in flüssigem Fluorwasserstoff eingesetzt. Die Elektrolyse findet vorteilhaft in einer Elektrolysezelle bei Temperaturen zwischen -25 und +10, vorzugsweise -5 und +5 °C, einer Anodenstromdichte von 2-30 mA/cm² und einer Zellspannung von 3 - 10, insbesondere 4 - 8 V, statt. Es kann sich als für den Elektrolyseprozess förderlich erweisen, durch ständige Umwälzung des Zellinhaltes den Elektrolyt dem Elektrodenbereich immer wieder zuzuführen.

Da die elektrochemische Fluorierung in flüssigem Fluorwasserstoff ein sehr energiereicher Prozeß ist, bei dem die Perfluorierung oft mit beträchtlichen Nebenreaktionen verbunden ist, war es nicht zu erwarten, daß ungesättigte bicyclische Verbindungen der Formel II sich auf dem Wege der elektrochemischen Fluorierung mit für eine zur praktischen Gewinnung der Verbindungen Ia ausreichenden Ausbeute perfluorieren lassen. Es mußte vielmehr mit Bruch an den C=C-Doppelbindungen des Cycloalkylenringes sowie auch mit Bruch von C-Heteroatom-Bindungen gerechnet werden. Die elektrochemische Perfluorierung der Verbindungen der Formel II gelingt erfindungsgemäß in zufriedenstellenden Ausbeuten, wenn in dem vorstehend angegebenen relativ niederen Temperaturbereich und bei der vorstehend angegebenen relativ niedrigen Zellspannung gearbeitet wird.

Zur weiteren Aufarbeitung wird das sich als schwere Phase am Boden der verwendeten Elektrolysezelle absetzende rohe Reaktionsprodukt abgetrennt und zur Zersetzung von allfälligen nur teilfluorierten Nebenprodukten einer Behandlung mit einer Alkali- oder Erdalkalimetallbase, insbesondere einem Alkali- oder Erdkalimetallhydroxid, in Gegenwart von Wasser und gegebenenfalls einem niederen aliphatischen primären oder sekundären Amin bei einer zur Zersetzung von nur partiell fluorierten Nebenprodukten ausreichend hohen Temperatur unterworfen. Diese Verfahrensstufe kann unter an sich bekannten Reaktionsbedingungen, beispielsweise analog zu den in den europäischen Patentanmeldungen Veröffentlichungs-Nr. 99 652 und 151 697 beschriebenen Methoden, erfolgen. Als zweckmäßig erweist sich das Behandeln des rohen Reaktionsproduktes mit einer 6- bis 10-normalen wäßrigen Alkalimetallhydroxidlösung, insbesondere Kaliumhydroxidlösung, und einem niederen aliphatischen Amin bei erhöhter Temperatur, vorzugsweise Siedetemperatur des Reaktionsgemisches. So kann das Reaktionsgemisch beispielsweise für eine Dauer von mehreren Stunden bis zu 8 Tagen unter Rückfluß zum Sieden erhitzt werden. Als niedere aliphatiche Amine eignen sich niedere bei Raumtemperatur flüssige primäre oder sekundäre Amine oder Diamine, vorzugsweise sekundäre Amine wie beispielsweise Dialkylamine mit bis zu 5 Kohlenstoffatomen in ihren Alkylresten oder Hexamethylendiamin oder auch cyclische Amine wie Piperidin. Vorzugsweise wird ein Dibutylamin wie beispielsweise Diisobutylamin eingesetzt.

Aus dem entstandenen Reaktionsgemisch können gebildete Gemische aus Verbindungen der Formel Ia und Ib auf an sich bekannte Weise, beispielsweise durch fraktionierte Destillation, isoliert werden. Die Gemische, in welchen im allgemeinen die Verbindungen der Formel Ia überwiegen, können gewünschtenfalls auf an sich bekannte Weise in die Verbindungen der Formeln Ia und Ib aufgetrennt werden.

Die durch fraktionierte Destillation aus dem Reaktionsgemisch abgetrennten Gemische sind frei von nicht perfluorierten Produkten. Sie können jedoch neben dem Hauptprodukt der Formel Ia noch ebenfalls perfluorierte und somit chemisch und physiologisch inerte Nebenprodukte enthalten. Insbesondere können sie solche perfluorierten Nebenprodukte enthalten, welche ein ähnliches Molekulargewicht wie das Hauptprodukt besitzen und damit im gleichen Temperaturbereich wie das Hauptprodukt sieden. Die Anwesenheit derartiger perfluorierter Nebenprodukte beeinträchtigt die bestimmungsgemäße Verwendung der Verbindungen jedoch nicht, so daß die destillativ gereinigten Produkte im allgemeinen ohne weitere Reinigung verwendet werden können.

Unter den Bedingungen der elektrochemischen Perfluorierung von Verbindungen der Formel II findet in piperidinringhaltigen Verbindungen teilweise in den Piperidinringen eine Ringverengung statt, so daß neben Verbindungen der Formeln Ia und Ib, worin X eine -CF₂-CF₂-CF₂-Gruppe bedeutet, in geringerem Ausmaß auch die dazu isomeren Verbindungen, worin X eine -CF(CF₃)-CF₂-Gruppe bedeutet, erhalten werden. Diese Isomerengemische von Verbindungen der Formel Ia und/oder Ib können in gleicher Weise wie reine Verbindungen der Formel Ia und/oder Ib verwendet werden.

Gewünschtenfalls können die Isomerengemische in ihre einzelnen Isomeren aufgetrennt werden. Zweckmäßigerweise werden eine Trennung von Gemischen der Verbindungen Ia und Ib und/oder eine Isomerentrennung mittels Adsorption/Desorption an Molekularsieben, vorzugsweise Molekularsieben mit einer Porengröße von 5-6 Å, durchgeführt. Als Molekularsiebe eignen sich z.B. anorganische Alumosilikate, Zeolithe und Silicalite Siliciumdioxide mit geeigneter Porengröße). Bevorzugt werden Zeolithe eingesetzt. Anorganische Molekularsiebe eignen sich generell zur Trennung von nur unsubstituierte perfluorierte Ringe enthaltenden Verbindungen von Perfluoroalkylsubstituenten enthaltenden Nebenprodukten. Je nach Größe der Perfluoroalkylsubstituenten werden hierfür Molekularsiebe mit Porengrößen zwischen 5 und 6,5 Å gewählt. Eine Auftrennung von Isomerengemischen kann auch durch präparative Gaschromatographie in an sich bekannter Weise erfolgen.

Die Ausgangsverbindungen der Formel II sind bekannt oder können nach an sich bekannten oder analog zu an sich bekannten Methoden hergestellt werden.

Die erfindungsgemäß hergestellten Verbindungen, insbesondere die Verbindung I′a oder deren Gemische mit Verbindung I′b, können zu medizinisch verwendbaren wäßrigen Emulsionen auf an sich bekannte Weise verarbeitet werden. Derartige Emulsionen stellen Emulsionen vom Öl-in-Wasser-Typ dar, welche 5 -50, insbesondere 15 - 25, vorzugsweise ca. 20 g, an perfluorierten Verbindungen, das ist an Verbindungen der Formel Ia oder Ib oder deren Gemischen und gegebenenfalls weiteren physiologisch geeigneten Perfluorcarbonen, pro 100 ml Emulsion und einen physiologisch verträglichen Emulgator und gegebenenfalls weitere physiologisch verträgliche Hilfsstoffe enthalten. Es kann sich als zweckmäßig erweisen, Perfluorcarbon-Gemische einzusetzen, welche einen geringeren Anteil an relativ hoch siedenden Verbindungen enthalten.

Als Emulgatoren eignen sich physiologisch verträgliche Emulgatoren, welche selbst untoxisch sind, keine Hämolyse verursachenden Eigenschaften haben und auch sonst keine Wechselwirkungen mit Komponenten des natürlichen Blutes eingehen und vollkommen aus dem Körper ausgeschieden oder unter Bildung untoxischer Metaboliten metabolisiert werden. Geeignete Emulgatoren sind zum Beispiel natürliche Phosphorlipide wie Eilecithine oder Sojalecithine, und Albumine. Ebenfalls geeignet sind physiologisch verträgliche nichtionische Emulgatoren vom Typ Athylenoxid-Propylenoxid-Mischpolymere, beispielsweise Mischpolymere mit einem Molekulargewicht im Bereich von 8000 -8500. Derartige Emulgatoren sind als Handelsprodukte erhältlich, beispielsweise unter dem Warenzeichen Pluronic^{R}, Hersteller Wyandotte Chemicals Corp. Die Emulgatoren können in den erfindungsgemäßen Emulsionen in einer Konzentration von beispielsweise 2 - 7 g pro 100 ml Emulsion enthalten sein. Außerdem können die Emulsionen noch weitere in Blutersatzmitteln übliche Hilfs- und Zusatzstoffe enthalten, beispielsweise Salze und Substanzen, welche zur Einstellung eines physiologisch verträglichen pH-Wertes, und/oder osmotischen und onkotischen Druckes dienen.

Die Emulsionen können in an sich bekannter Weise mit. üblichen Emulsionstechniken hergestellt werden. So kann die Emulgierung durch Ultraschall- und/oder Hochdruckhomogenisation erfolgen.

Die Emulsionen können in der Medizin Anwendung finden als Sauerstoff transportierende Blutersatzmittel. Außerdem sind sie als Sauerstoff transportierende Perfusionslösungen, beispielsweise zum Schutze von freigelegten Organen in der Chirurgie, beispielsweise zum Schutze des Myokardium gegen Hypoxie in der Herzchirurgie, einsetzbar. Ferner können die Emulsionen auch als Hilfsmittel in der Diagnostik, beispielsweise für die Ultrasonographie und ¹⁹F-NMR-Tomographie Einsatz finden. Auch in der Biotechnologie können derartige Emulsionen in Sauerstoff übertragenden Nährmedien, beispielsweise zur Züchtung von tierischen und pflanzlichen Zellen oder bei der Interferonsynthese Anwendung finden.

Die Verbindungen der Formel Ia, Ib oder deren Gemische können auch in anderen Gebieten der Technik, in welchen flüssige oder wachsartige Substanzen welche chemisch inert sind und/oder ein Lösungsvermögen für Gase aufweisen, benötigt werden, eingesetzt werden. So eignen sich die Verbindungen und ihre Gemische beispielsweise als inerte Kühlmittel, Schmiermittel, Sperr- und Hydraulikflüssigkeiten, Isoliermedien in der Elektrotechnik, sowie Mittel zum Dampfphasenlöten bzw. als Zusätze zu Mitteln für die vorgenannten Zwecke. Aufgrund ihres Lösevermögens für Gase eignen sich die Verbindungen und ihre Gemische als inertes Medium für die Diffusion von Gasen zwischen verschiedenen Phasen. Damit können die Verbindungen auch Verwendung finden bei Verfahren zur technischen Trennung von Gasen, beispielsweise der Dialysetrennung von Gasen, wobei die Verbindungen als inerte Austauschphase dienen.

Die nachstehenden Beispiele sollen die Erfindung näher erläutern ohne jedoch ihren Umfang zu beschränken.

Als Elektrolysezellen für die elektrochemische Fluorierung werden Zellen mit Elektroden aus Nickel verwendet, welche entweder ein Fassungsvolumen von 300 ml und eine Anodenfläche von 475 cm² oder ein Fassungsvolumen von 960 ml und eine Anodenfläche von 1530 cm² besitzen. Die Zellen sind mit einem Rückflußkühler versehen, welcher auf einer Temperatur zwischen -15 und -20 °C gehalten wird.

### Beispiel 1:

Herstellung von einem Gemisch aus Perfluorocyclohexylmorpholin und Perfluoro-n-hexylmorpholin.

Eine 5- bis 15%ige Lösung von Morpholincyclohexen-(1) in vorgetrocknetem gekühlten flüssigen Fluorwasserstoff wurde in einer Elektrolysezelle bei einer Anodenstromdichte von 3 bis 20 mA/cm² einer Zellspannung von 5 bis 6,5 V und einer Zelltemperatur von -8 bis +5 °C perfluoriert. Von Zeit zu Zeit wurden weiteres in flüssigem Fluorwasserstoff gelöstes Morpholincyclohexen-(1) und verbrauchter Fluorwasserstoff nachgefüllt um ein kontinuierliches Arbeiten der Zelle zu ermöglichen. Die sich am Zellenboden sammelnde, das rohe Reaktionsprodukt enthaltende schwere Phase wurde von Zeit zu Zeit abgelassen. Das Rohprodukt wurde mit jeweils den gleichen Volumina einer wäßrigen 8-N Kaliumhydroxidlösung und Dibutylamin versetzt. Das Gemisch wurde 8 Tage am Rückfluß erhitzt. Anschließend wurde das Gemisch fraktionierend destilliert. Bei der Destillation erhielt man eine Hauptfraktion im Siedebereich von 145 bis 148 °C, welche hauptsächlich aus perfluoriertem Cyclohexylmorpholin bestand und ohne weitere Reinigung für die meisten der in der vorstehenden Beschreibung angegebenen Verwendungszwecke eingesetzt werden kann. Die Ausbeute betrug 30 %. Gaschromatographische Analyse ergab, daß das Destillat ein Gemisch aus 65 % des Hauptproduktes Perfluorocyclohexylmorpholin, 33 % des Nebenproduktes Perfluoro-n-hexylmorpholin und 2 % weiteren Nebenprodukten darstellte mit einem Siedepunkt von 147,5 - 148,5 °C und einem Dampfdruck bei 37 °C von 12 Torr.

### Beispiel 2:

Herstellung von einem Gemisch aus Perfluorcyclohexylmorpholin und Perfluoro-n-hexylmorpholin.

Es wurde wie in Beispiel 1 beschrieben gearbeitet, wobei jedoch eine zusätzlich mit einer Umwälzpumpe versehene Elektrolysezelle eingesetzt wurde. Durch ständige Umwälzung des gesamten Zellinhaltes wurde während des Elektrolyseprozesses der Elektrolyt immer wieder dem Elektrodenbereich zugeführt. Zum kontinuierlichen Betrieb wurde von Zeit zu Zeit wie in Beispiel 1 beschrieben, weiteres Ausgangsprodukt zugeführt. Der Elektrolyseprozess wurde durch Überwachung des Strom-Spannungs-Verlauf kontrolliert. Von Zeit zu Zeit, wenn sich durch einen starken Abfall der Leitfähigkeit ein Anwachsen der Konzentration an perfluoriertem Produkt und dementsprechend ein Abfall der Elektrolytkonzentration anzeigte, wurde die Umwälzung unterbrochen und die sich am Zellboden ansammelnde das Rohprodukt enthaltende schwere Phase abgelassen. Das Rohprodukt wurde wie in Beispiel 1 beschrieben aufgearbeitet. Das als Destillat erhaltene Gemisch hatte die in Beispiel 1 angegebene Zusammensetzung. Es wurde eine Ausbeute von 43 % erzielt.

### Beispiel 3:

Herstellung von reinem Perfluorocyclohexylmorpholin durch Auftrennung des in Beispiel 1 oder 2 erhaltenen Gemisches.

Auf eine mit einem zeolithischen Molekularsieb der Porengröße 5 Å gefüllte Glassäule, durch die ein konstanter Strom Helium mit einer strömungsgeschwindigkeit von ml/min floß, wurde bei 120 °C über einen Verdampfer das in Beispiel 1 erhaltene Gemisch aus Perfluorocyclohexylmorpholin und Perfluoro-n-hexyl-morpholin aufgegeben. Während das Perfluorcyclohexylmorpholin die Säule passierte, wurde das den C₆F₁₃-Rest enthaltende Nebenprodukt weitgehend zurückgehalten. Es wurde so reines perfluoriertes Cyclohexylmorpholin mit einem Siedepunkt von 147,5 -149,5 °C gewonnen. Nachdem alles Perfluorcyclohexylmorpholin und überschüssiges Perfluor-n-hexylmorpholin die Säule verlassen hatten, wurde die Temperatur auf 300 °C gesteigert. In einer Kühlfalle am Ausgang der Säule kondensierte reines Perfluor-n-hexylmorpholin mit einem Siedepunkt von 149 - 150 °C.

Das Perfluorocyclohexylmorpholin und das Perfluoro-n-hexylmorpholin wiesen bei 37 °C einen Dampfdruck von 12 Torr auf.

Für das Perfluorocyclohexylmorpholin und das Perfluoro-n-hexylmorpholin wurden das Sauerstofflösevermögen (=O₂-lös.) und die kritische Lösungstemperatur in n-Hexan (=CTSH), welche als gutes Indiz für die Verweildauer der Perfluorocarbone im Körper angesehen werden kann, nach folgenden Methoden bestimmt.

### A. Bestimmung der Sauerstofflösekapazität.

Die Bestimmung wurde in einem mit einem Ventil für Sauerstoffzufuhr, einem Ventil zur Evakuierung und einem Druckmesser versehenen 2 l-Rundkolben mit einem zur Aufnahme des zu untersuchenden Perfluorcarbon geeigneten unteren Fortsatz, in welchen das Perfluocarbon durch ein Septum eingeführt werden kann, durchgeführt.

Die Apparatur wurde zunächst mit einer Wasserstrahlpumpe evakuiert, mit Sauerstoff gefüllt, nochmals evakuiert und wieder mit Sauerstoff bis zum Erreichen eines Innendruckes von 1013 mbar gefüllt. Sodann wurde das Perfluorcarbon durch ein Septum in den unteren Fortsatz eingeführt und dort 2 Stunden unter Sauerstoffatmosphäre gerührt. Die Temperatur wurde mit Hilfe eines Kryostaten auf 37 °C gehalten. Nach 2 Stunden wurde ein Teil des Perfluorcarbon entnommen und gaschromatographisch auf seinen Sauerstoffgehalt untersucht.

Die gaschromatische Untersuchung wurde mit einem Gaschromatographen CAP 12 der Fa. Gira, Frankreich, vorgenommen. Als Trägergas wurde Helium mit einem Fluß von 20 ml/min. eingesetzt. Als Trennsäule wurde eine Glassäule 2 m x 4 mm ⌀ gefüllt, mit Molekularsieb 5 Å, 40-60 mesh, verwendet. Die Ofentemperatur betrug 100 °C, die Injektortemperatur 250 °C und die Detektortemperatur 200 °C. Das Probenvolumen betrug 30 - 50 »l.

### B. Bestimmung der kritischen Lösungstemperatur in n-Hexan.

Gleiche Volumina (jeweils 50 »l) des Perfluorcarbons und des n-Hexan wurden in ein Glasrohr (ca. 3 cm lang ⌀ 3mm) unter Kühlung (-70 °C) eingeschmolzen. Um die Mischbarkeit der beiden Flüssigkeiten zu bestimmen, wurde in einer Schmelzpunktbestimmungsapparatur die Badtemperatur langsam erhöht bzw. erniedrigt und die Temperatur gemessen, bei der die zwei Phasen vermischen bzw. sichtbar werden.

| Ergebnisse: | O₂-lös. bei 37 °C | CTSH |
|---|---|---|
| Perfluorocyclohexylmorpholin | 47 Vol % | 31 °C |
| Perfluoro-n-hexylmorpholin | 45 Vol % | 45 °C |

Die vorstehenden Ergebnisse zeigen, daß die Verbindungen als Sauerstoff übertragende Perfluorcarbonc geeignet sind, und daß das Perfluorocyclohexylmorpholin eine wesentlich niedrigere CTSH als das Perfluoro-n-hexylmorpholin aufweist. Diese ist ein Indiz für eine zur Anwendung in Blutersatzmitteln gut geeignete Verweilhalbwertzeit im Körper.

Analog zu den in den vorstehenden Beispielen angegebenen Methoden können auch Perfluorocyclopentylmorpholin bzw. Perfluorocyclopentylmorpholin/Perfluoro-n-pentylmorpholinGemische und Perfluorocyclohexylpiperidin bzw. Perfluorocyclohexylpiperidin/Perfluoro-n-hexylpiperidin-Gemische durch elektrochemische Perfluorierung entsprechender Ausgangsverbindungen der Formel II in guten Ausbeuten, beispielsweise von über 40 %, erhalten werden. Die verwendeten Ausgangsstoffe der Formel II können analog der nachfolgend für die Herstellung von Morpholinocyclohexen-(1) angegebenen Arbeitsvorschrift hergestellt werden.

### Herstellung von Morpholinocyclohexen-(1):

1,2 Mol Morpholin werden mit 1,0 Mol Cyclohexanon und 200 ml Toluol solange am Wasserabscheider erhitzt, bis kein Reaktionswasser mehr abgeschieden wird. Anschließend wird die Verbindung der Formel II durch fraktionierte Destillation aus dem Reaktionsgemisch isoliert.

### Beispiel I:

Herstellung einer perfluoriertes Cyclohexylmorpholin enthaltenden wässrigen Emulsion.

2 g des in Beispiel 2 erhaltenen perfluorierten Cyclo-hexylmorpholins wurden zu 0,3 g des Emulgators Pluronic^{R}F68 (=Ethylenoxid/Propylenoxid-Blockpolymeres, mittleres Molekulargewicht 8300, Hersteller Firma Wyandotte) gegeben und das Gemisch wurde mit Wasser auf ein Gesamtvolumen von 10 ml aufgefüllt. Es wurde 2 Minuten mit Ultraschall homogenisiert. Die entstandene Emulsion hatte eine Kolloidteilchengröße von ca. 200 nm, welche bei Lagerung bei +4 °C über mehrere Wochen konstant blieb.

### Beispiel II:

Eine als Blutersatzmittel oder Perfusionsmedium geeignete perfluoriertes Cyclohexylmorpholin enthaltende wässrige Emulsion.

| Zusammensetzung: | |
|---|---|
| Perfluoriertes Cyclohexylmorpholin erhalten gemäß Beispiel 2 | 200 g/l |
| Emulgator (Pluronic^{R} F68) | 27 g/l |
| Glycerin | 8 g/l |
| Eiweißphospholipide | 4 g/l |
| Hydroxyäthylstärke (mittleres Molekulargewicht 200000) | 30 g/l |
| Na⁺ | 128,0 mMol/l |
| K⁺ | 4,5 mMol/l |
| Ca⁺⁺ | 2,5 mMol/l |
| Mg⁺⁺ | 2,1 mMol/l |
| Cl⁻ | 116,0 mMol/l |
| HCO₃⁻ | 25 mMol/l |
| Glucose | 10,0 mMol/l |
| Steriles destilliertes Wasser | ad 1 l |

Die vorstehend angegebenen Bestandteile wurden in an sich bekannter Weise homogenisiert.

## Patentansprüche (Patentansprüche für folgende(n) Vertragsstaat(en): AT, BE, CH, DE, DK, FR, GB, IT, NL, SE)

1. Verfahren zur Herstellung von Verbindungen der allgemeinen Formeln Ia und Ib 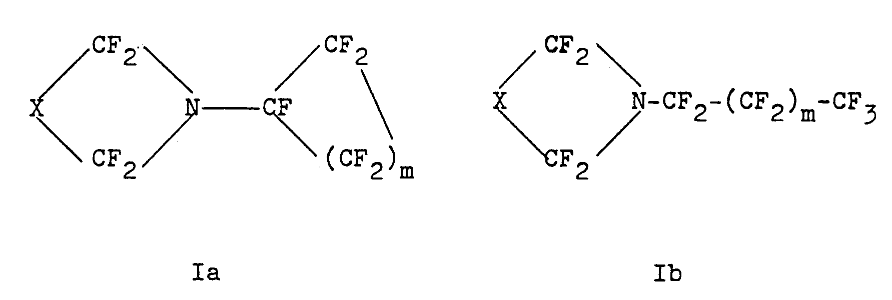 worin m 3 oder 4 bedeutet und X eine -CF₂-O-CF₂-Gruppe, eine -CF₂-CF₂-CF₂-Gruppe oder eine -CF(CF₃)-CF₂-Gruppe darstellt,
oder deren Gemischen, dadurch gekennzeichnet, daß man
a) eine Lösung von Verbindungen der allgemeinen Formel II 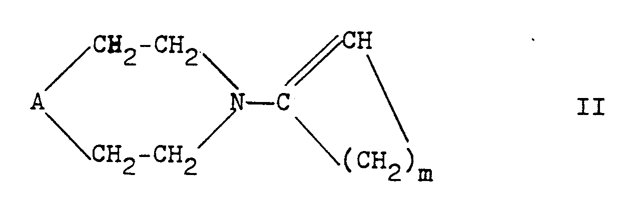 worin m obige Bedeutung besitzt und A Sauerstoff oder eine -CH₂-Gruppe bedeutet, in flüssigem Fluorwasserstoff elektrolysiert und ein die perfluorierten Verbindungen der Formel Ia und Ib neben nur partiell fluorierten Nebenprodukten enthaltendes rohes Reaktionsprodukt abtrennt,
b) das rohe Reaktionsprodukt mit einer Alkali- oder Erdalkalimetallbase, insbesondere einem Alkali- oder Erdalkalimetallhydroxid, in Gegenwart von Wasser und gegebenenfalls einem niederen aliphatischen primären oder sekundären Amin bei einer zur Zersetzung von nur partiell fluorierten Nebenprodukten ausreichend hohen Temperatur behandelt,
c) ein Gemisch der Formeln Ia und Ib aus einem in Verfahrensstufe b) erhaltenem Reaktionsgemisch isoliert und dieses gewünschtenfalls in die Verbindungen der Formel Ia und Ib auftrennt, und
d) gewünschtenfalls erhaltene Isomerengemische aus Verbindungen der Formeln Ia oder Ib, worin X eine -CF₂-CF₂-CF₂-Gruppe bedeutet und m obige Bedeutung besitzt und dazu isomeren Verbindungen der Formeln Ia oder Ib, worin X eine -CF(CF₃)-CF₂-Gruppe bedeutet, in die einzelnen Isomeren auftrennt.

2. Verfahren nach Anspruch 1, dadurch gekennzeichnet, daß als Base in Stufe b) eine wäßrige Alkalimetallhydroxidlösung, insbesondere Kaliumhydroxidlösung eingesetzt wird.

3. Verfahren nach Anspruch 2, dadurch gekennzeichnet, daß in Stufe b) eine wäßrige Alkalimetallhydroxidlösung und ein niederes aliphatisches primäres oder sekundäres Amin, vorzugsweise ein sekundäres Amin, insbesondere ein Dibutylamin, eingesetzt werden.

4. Verfahren nach Anspruch 1, dadurch gekennzeichnet, daß die Elektrolyse in Stufe a) bei einer Temperatur zwischen -25 und +10 °C, insbesondere -5 und +5 °C, und bei einer Zellspannung von 3-10, insbesondere 4-8, V durchgeführt wird.

5. Verfahren nach Anspruch 1, dadurch gekennzeichnet, daß in Stufe c) die Verbindungen der Formel Ia und Ib oder deren Gemische destillativ aus dem Reaktionsgemisch isoliert werden.

6. Verfahren nach Anspruch 1, dadurch gekennzeichnet, daß in Stufe c) oder d) das Gemisch mittels Adsorption/Desorption an Molekularsieben aufgetrennt wird.

7. Verfahren nach Anspruch 6, dadurch gekennzeichnet, daß anorganische Molekularsiebe mit einer Porengroße von 5 bis 6,5 A eingesetzt werden.

8. Verwendung von Perfluoro-N-cyclohexylmorpholin der Formel I'a oder dessen Gemisch mit Perfluoro-N-n-hexylmorpholin der Formel I'b 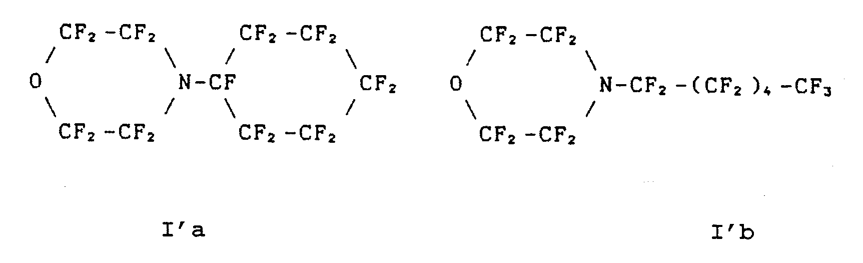 als sauerstoffübertragender Bestandteil von wäßrigen, zum Sauerstofftransport befähigten, medizinisch verwendbaren Perfluorcarbonemulsionen.

9. Nach dem Verfahren des Anspruchs 1 erhältliches Gemisch von Perfluoro-N-cyclohexylmorpholin der Formel I'a mit Perfluoro-N-n-hexylmorpholin der Formel I'b.

## Patentansprüche (Patentansprüche für folgende(n) Vertragsstaat(en): ES)

1. Verfahren zur Herstellung von Verbindungen der allgemeinen Formeln Ia und Ib 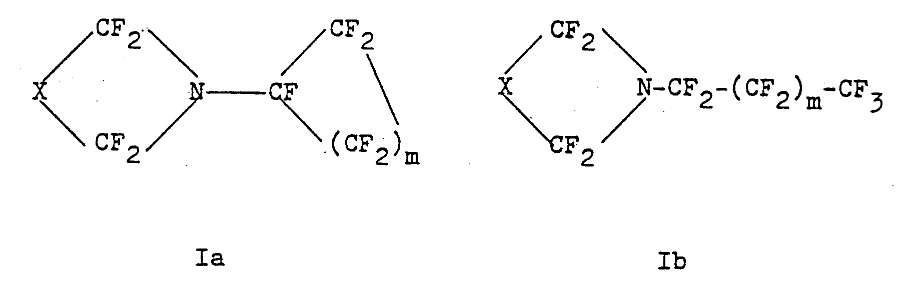 worin m 3 oder 4 bedeutet und X eine -CF₂-O-CF₂-Gruppe, eine -CF₂-CF₂-CF₂-Gruppe oder eine -CF(CF₃)-CF₂-Gruppe darstellt,
oder deren Gemischen, dadurch gekennzeichnet, daß man
a) eine Lösung von Verbindungen der allgemeinen Formel II 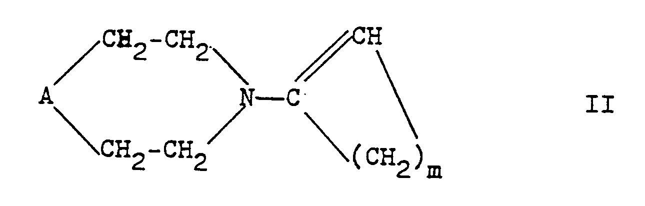 worin m obige Bedeutung besitzt und A Sauerstoff oder eine -CH₂-Gruppe bedeutet, in flüssigem Fluorwasserstoff elektrolysiert und ein die perfluorierten Verbindungen der Formel Ia und Ib neben nur partiell fluorierten Nebenprodukten enthaltendes rohes Reaktionsprodukt abtrennt,
b) das rohe Reaktionsprodukt mit einer Alkali- oder Erdalkalimetallbase, insbesondere einem Alkali- oder Erdalkalimetallhydroxid, in Gegenwart von Wasser und gegebenenfalls einem niederen aliphatischen primären oder sekundären Amin bei einer zur Zersetzung von nur partiell fluorierten Nebenprodukten ausreichend hohen Temperatur behandelt,
c) ein Gemisch der Formeln Ia und Ib aus einem in Verfahrensstufe b) erhaltenem Reaktionsgemisch isoliert und dieses gewünschtenfalls in die Verbindungen der Formel Ia und Ib auftrennt, und
d) gewünschtenfalls erhaltene Isomerengemische aus Verbindungen der Formeln Ia oder Ib, worin X eine -CF₂-CF₂-CF₂-Gruppe bedeutet und m obige Bedeutung besitzt und dazu isomeren Verbindungen der Formeln Ia oder Ib, worin X eine -CF(CF₃)-CF₂-Gruppe bedeutet, in die einzelnen Isomeren auftrennt.

2. Verfahren nach Anspruch 1, dadurch gekennzeichnet, daß als Base in Stufe b) eine wäßrige Alkalimetallhydroxidlösung, insbesondere Kaliumhydroxidlösung eingesetzt wird.

3. Verfahren nach Anspruch 2, dadurch gekennzeichnet, daß in Stufe b) eine wäßrige Alkalimetallhydroxidlösung und ein niederes aliphatisches primäres oder sekundäres Amin, vorzugsweise ein sekundäres Amin, insbesondere ein Dibutylamin, eingesetzt werden.

4. Verfahren nach Anspruch 1, dadurch gekennzeichnet, daß die Elektrolyse in Stufe a) bei einer Temperatur zwischen -25 und +10 °C, insbesondere -5 und +5 °C, und bei einer Zellspannung von 3-10, insbesondere 4-8, V durchgeführt wird.

5. Verfahren nach Anspruch 1, dadurch gekennzeichnet, daß in Stufe c) die Verbindungen der Formel Ia und Ib oder deren Gemische destillativ aus dem Reaktionsgemisch isoliert werden.

6. Verfahren nach Anspruch 1, dadurch gekennzeichnet, daß in Stufe c) oder d) das Gemisch mittels Adsorption/Desorption an Molekularsieben aufgetrennt wird.

7. Verfahren nach Anspruch 6, dadurch gekennzeichnet, daß anorganische Molekularsiebe mit einer Porengröße von 5 bis 6,5 Å eingesetzt werden.

8. Verfahren zur Herstellung von wäßrigen, zum Sauerstofftransport befähigten, medizinisch verwendbaren Perfluorcarbonemulsionen, dadurch gekennzeichnet, daß man als sauerstoffübertragenden Bestandteil Perfluoro-N-cyclohexylmorpholin der Formel I'a oder dessen Gemisch mit Perfluoro-N-n-hexylmorpholin der Formel I'b 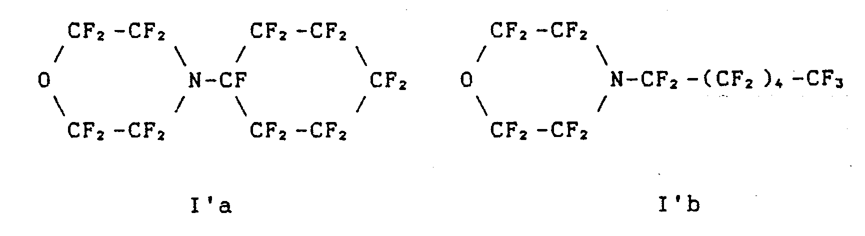 einsetzt.

## Claims (Claims for the following Contracting State(s): AT, BE, CH, DE, DK, FR, GB, IT, NL, SE)

1. A method for the preparation of compounds of the general formulae Ia and Ib, 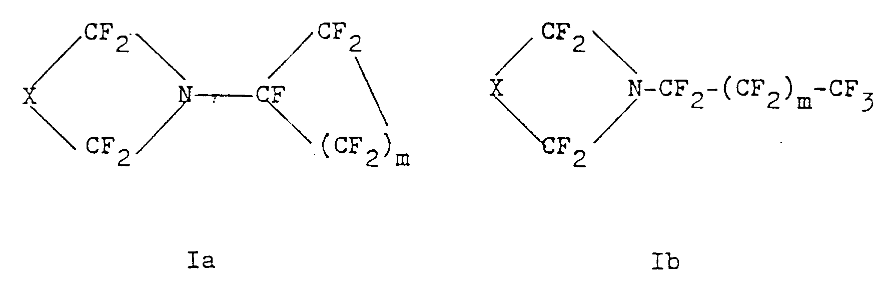 wherein m is 3 or 4 and X is a -CF₂-O-CF₂- group, a -CF₂-CF₂-CF₂- group or a -CF(CF₃)-CF₂- group,
or mixtures thereof, characterised in that
a) a solution of compounds of the general formula II, 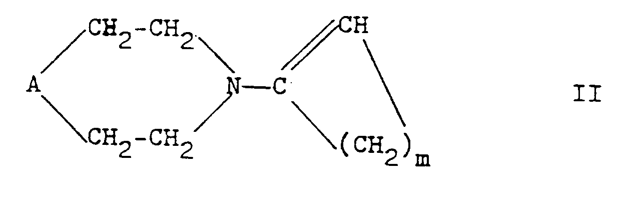 wherein m has the above meaning and A is oxygen or a -CH₂- group, in liquid hydrogen fluoride is electrolysed and a crude reaction product containing the perfluorinated compounds of Formula Ia and Ib in addition to only partially fluorinated byproducts is separated off,
b) the crude reaction product is treated with an alkali metal base or alkaline earth metal base, in particular an alkali metal hydroxide or alkaline earth metal hydroxide, in the presence of water and optionally a lower aliphatic primary or secondary amine at a temperature sufficiently high for the decomposition of only partially fluorinated byproducts,
c) a mixture of Formulae Ia and Ib is isolated from a reaction mixture obtained in process step b) and said mixture is separated into the compounds of Formula Ia and Ib if desired, and
d) if desired resulting isomer mixtures of compounds of Formulae Ia or Ib, wherein X is a -CF₂-CF₂-CF₂-group and m has the above meaning, and compounds of Formula Ia or Ib which are isomeric thereto, wherein X is a -CF(CF₃)-CF₂- group, are separated into the individual isomers.

2. A method according to Claim 1, characterised in that an aqueous alkali metal hydroxide solution, in particular potassium hydroxide solution, is used as the base in stage b).

3. A method according to Claim 2, characterised in that an aqueous alkali metal hydroxide solution and a lower aliphatic primary or secondary amine, preferably a secondary amine, in particular a dibutylamine, are used in stage b).

4. A method according to Claim 1, characterised in that the electrolysis in stage a) is performed at a temperature between -25 and +10°C, in particular -5 and +5°C, and at a cell voltage of 3-10, in particular 4-8, volts.

5. A method according to Claim 1, characterised in that in stage c) the compounds of Formula Ia and Ib or mixtures thereof are isolated from the reaction mixture by distillation.

6. A method according to Claim 1, characterised in that in stage c) or d) the mixture is separated by means of adsorption/desorption on molecular sieves.

7. A method according to Claim 6, characterised in that inorganic molecular sieves having a pore size of 5 to 6.5 Å are used.

8. The use of perfluoro-N-cyclohexylmorpholine of Formula I'a or the mixture thereof with perfluoro-N-n-hexylmorpholine of Formula I'b 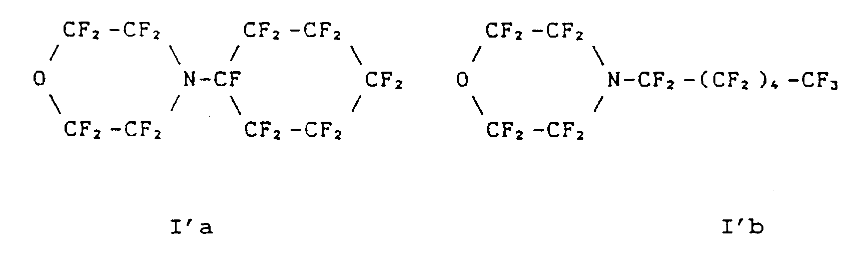 as an oxygen-transferring constituent of aqueous medically usable perfluorocarbon emulsions which are capable of transferring oxygen.

9. A mixture of perfluoro-N-cyclohexylmorpholine of Formula I'a with perfluoro-N-n-hexylmorpholine of Formula I'b, obtainable according to the method of Claim 1.

## Claims (Claims for the following Contracting State(s): ES)

1. A method for the preparation of compounds of the general formulae Ia and Ib, 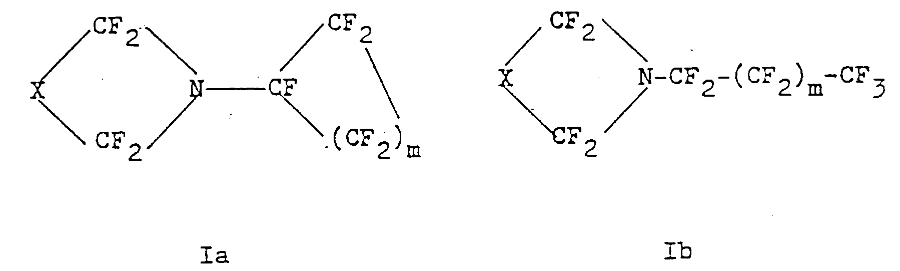 wherein m is 3 or 4 and X is a -CF₂-O-CF₂- group, a -CF₂-CF₂-CF₂- group or a -CF(CF₃)-CF₂- group,
or mixtures thereof, characterised in that
a) a solution of compounds of the general formula II, 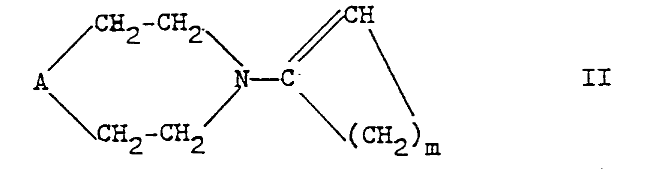 wherein m has the above meaning and A is oxygen or a -CH₂- group, in liquid hydrogen fluoride is electrolysed and a crude reaction product containing the perfluorinated compounds of Formula Ia and Ib in addition to only partially fluorinated byproducts is separated off,
b) the crude reaction product is treated with an alkali metal base or alkaline earth metal base, in particular an alkali metal hydroxide or alkaline earth metal hydroxide, in the presence of water and optionally a lower aliphatic primary or secondary amine at a temperature sufficiently high for the decomposition of only partially fluorinated byproducts,
c) a mixture of Formulae Ia and Ib is isolated from a reaction mixture obtained in process step b) and said mixture is separated into the compounds of Formula Ia and Ib if desired, and
d) if desired resulting isomer mixtures of compounds of Formulae Ia or Ib, wherein X is a -CF₂-CF₂-CF₂-group and m has the above meaning, and compounds of Formula Ia or Ib which are isomeric thereto, wherein X is a -CF(CF₃)-CF₂- group, are separated into the individual isomers.

2. A method according to Claim 1, characterised in that an aqueous alkali metal hydroxide solution, in particular potassium hydroxide solution, is used as the base in stage b).

3. A method according to Claim 2, characterised in that an aqueous alkali metal hydroxide solution and a lower aliphatic primary or secondary amine, preferably a secondary amine, in particular a dibutylamine, are used in stage b).

4. A method according to Claim 1, characterised in that the electrolysis in stage a) is performed at a temperature between -25 and +10°C, in particular -5 and +5°C, and at a cell voltage of 3-10, in particular 4-8, volts.

5. A method according to Claim 1, characterised in that in stage c) the compounds of Formula Ia and Ib or mixtures thereof are isolated from the reaction mixture by distillation.

6. A method according to Claim 1, characterised in that in stage c) or d) the mixture is separated by means of adsorption/desorption on molecular sieves.

7. A method according to Claim 6, characterised in that inorganic molecular sieves having a pore size of 5 to 6.5 Å are used.

8. A method for the preparation of aqueous medically usable perfluorocarbon emulsions which are capable of transferring oxygen, characterised in that perfluoro-N-cyclohexylmorpholine of Formula I'a or the mixture thereof with perfluoro-N-n-hexylmorpholine of Formula I'b 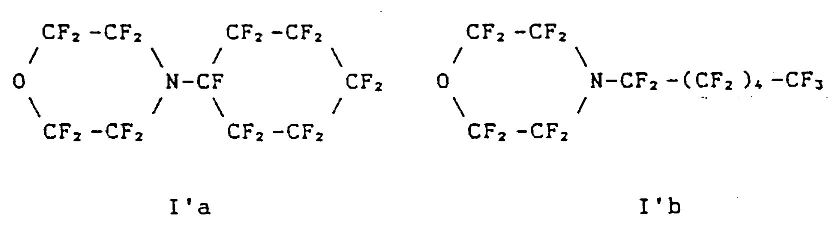 is used as an oxygen-transferring constituent.

## Revendications (Revendications pour l'(les) Etat(s) contractant(s) suivant(s): AT, BE, CH, DE, DK, FR, GB, IT, NL, SE)

1. Procédé pour préparer des composés de formules générales Ia et Ib 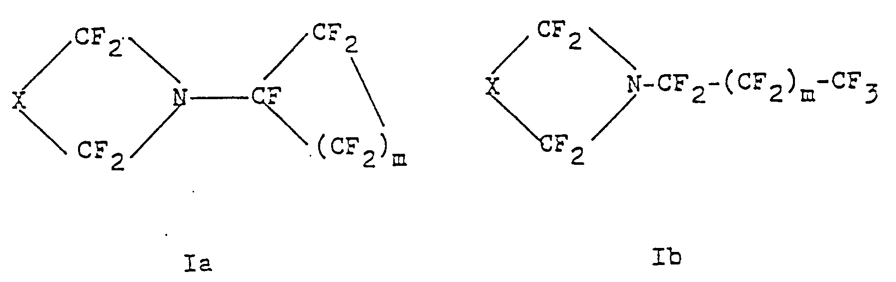 dans lesquelles m vaut 3 ou 4 et X est un groupe -CF₂-O-CF₂, un groupe -CF₂-CF₂-CF₂- ou un groupe -CF(CF₃)-CF₂-,
ou leurs mélanges, caractérisé en ce que
a) on électrolyse une solution de composés de formule générale II, 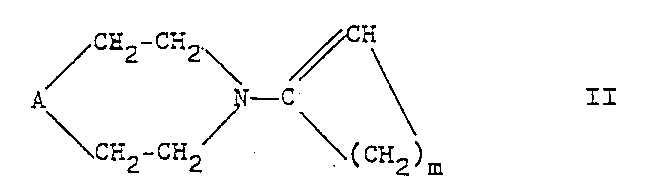 dans laquelle m a les significations données ci-dessus et A est un oxygène ou un groupe -CH₂-, dans de l'acide fluorhydrique liquide, et on sépare un produit de réaction brut, qui, outre des produits secondaires seulement partiellement fluorés, contient les composés perfluorés de formules Ia et Ib,
b) à une température suffisamment haute pour la décomposition de produits secondaires seulement partiellement fluorés, et en présence d'eau et éventuellement d'une amine aliphatique primaire ou secondaire inférieure, on traite le produit de réaction brut avec une base d'un métal alcalin ou alcalino-terreux, en particulier un hydroxyde d'un métal alcalin ou alcalino-terreux,
c) on isole un mélange de produits de formules Ia et Ib, à partir d'un mélange réactionnel obtenu dans l'étape b), et, si on le souhaite, on le sépare en les composés de formules Ia et Ib, et
d) on sépare éventuellement en les différents isomères les mélanges d'isomères obtenus, constitués de composés de formules Ia ou Ib, où X est un groupe -CF₂-CF₂-CF₂- et m a les significations ci-dessus, et de composés de formules Ia ou Ib, isomères des précédents et dans lesquels X est un groupe -CF(CF₃)-CF₂-.

2. Procédé selon la revendication 1, caractérisé en ce qu'on utilise comme base pour l'étape b) une solution aqueuse d'un hydroxyde d'un métal alcalin, en particulier une solution d'hydroxyde de potassium.

3. Procédé selon la revendication 2, caractérisé en ce que, dans l'étape b), on utilise une solution aqueuse d'un hydroxyde d'un métal alcalin et une amine aliphatique inférieure primaire ou secondaire, de préférence une amine secondaire, en particulier une dibutylamine.

4. Procédé selon la revendication 1, caractérisé en ce que l'électrolyse de l'étape a) est mise en oeuvre à une température de -25 à +10 et en particulier de -5 à +5°C, et à une tension de cellule de 3 à 10 et en particulier de 4 à 8 V.

5. Procédé selon la revendication 1, caractérisé en ce que, dans l'étape c), les composés de formules Ia et Ib ou leurs mélanges sont isolés du mélange réactionnel par distillation.

6. Procédé selon la revendication 1, caractérisé en ce que, dans l'étape c) ou d), le mélange est séparé par adsorption/désorption sur tamis moléculaires.

7. Procédé selon la revendication 6, caractérisé en ce qu'on utilise un tamis moléculaire minéral ayant une taille de pores de 5 à 6,5 A.

8. Utilisation de la perfluoro-N-cyclohexylmorpholine de formule I'a ou de son mélange avec la perfluoro-N-n-hexylmorpholine de formule I'b 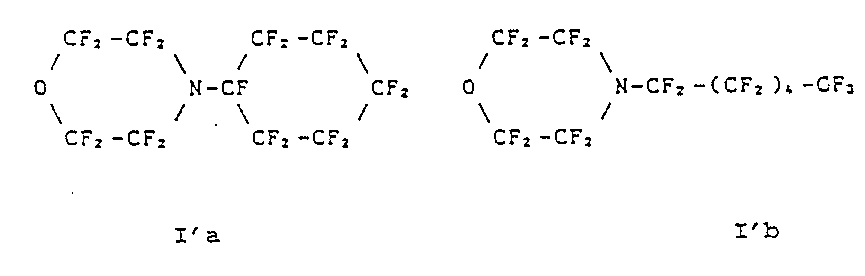 comme constituant de transfert d'oxygène dans des émulsions aqueuses de perfluorocarbones susceptibles d'assurer le transport de l'oxygène et utilisables en médecine.

9. Mélange, obtenu par le procédé selon la revendication 1, de perfluoro-N-cyclohexylmorpholine de formule I'a et de perfluoro-N-n-hexylmorpholine de formule I'b.

## Revendications (Revendications pour l'(les) Etat(s) contractant(s) suivant(s): ES)

1. Procédé pour préparer des composés de formules générales Ia et Ib 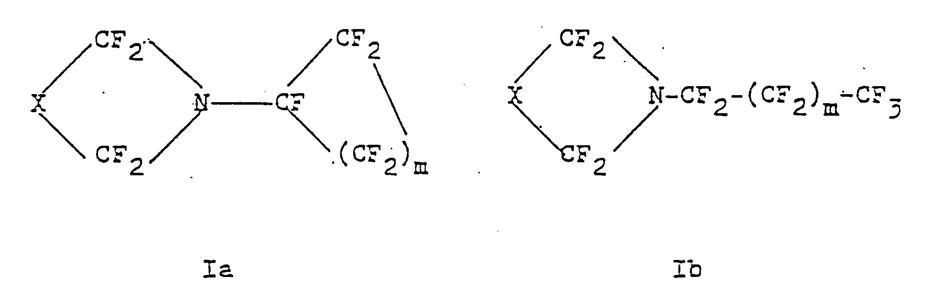 dans lesquelles m vaut 3 ou 4 et X est un groupe -CF₂-O-CF₂, un groupe -CF₂-CF₂-CF₂- ou un groupe -CF(CF₃)-CF₂-,
ou leurs mélanges, caractérisé en ce que
a) on électrolyse une solution de composés de formule générale II, 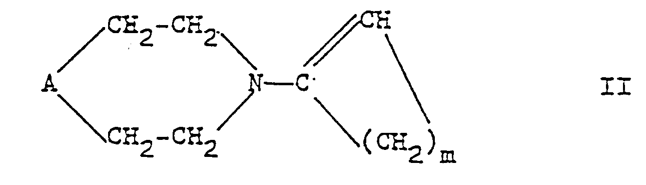 dans laquelle m a les significations données ci-dessus et A est un oxygène ou un groupe -CH₂-, dans de l'acide fluorhydrique liquide, et on sépare un produit de réaction brut, qui, outre des produits secondaires seulement partiellement fluorés, contient les composés perfluorés de formules Ia et Ib,
b) à une température suffisamment haute pour la décomposition de produits secondaires seulement partiellement fluorés, et en présence d'eau et éventuellement d'une amine aliphatique primaire ou secondaire inférieure, on traite le produit de réaction brut avec une base d'un métal alcalin ou alcalino-terreux, en particulier un hydroxyde d'un métal alcalin ou alcalino-terreux,
c) on isole un mélange de produits de formules Ia et Ib, à partir d'un mélange réactionnel obtenu dans l'étape b), et, si on le souhaite, on le sépare en les composés de formules Ia et Ib, et
d) on sépare éventuellement en les différents isomères les mélanges d'isomères obtenus, constitués de composés de formules Ia ou Ib, où X est un groupe -CF₂-CF₂-CF₂- et m a les significations ci-dessus, et de composés de formules Ia ou Ib, isomères des précédents et dans lesquels X est un groupe -CF(CF₃)-CF₂-.

2. Procédé selon la revendication 1, caractérisé en ce qu'on utilise comme base pour l'étape b) une solution aqueuse d'un hydroxyde d'un métal alcalin, en particulier une solution d'hydroxyde de potassium.

3. Procédé selon la revendication 2, caractérisé en ce que, dans l'étape b), on utilise une solution aqueuse d'un hydroxyde d'un métal alcalin et une amine aliphatique inférieure primaire ou secondaire, de préférence une amine secondaire, en particulier une dibutylamine.

4. Procédé selon la revendication 1, caractérisé en ce que l'électrolyse de l'étape a) est mise en oeuvre à une température de -25 à +10 et en particulier de -5 à +5°C, pour une tension de cellule de 3 à 10 et en particulier de 4 à 8 V.

5. Procédé selon la revendication 1, caractérisé en ce que, dans l'étape c), les composés de formules Ia et Ib ou leurs mélanges sont isolés du mélange réactionnel par distillation.

6. Procédé selon la revendication 1, caractérisé en ce que, dans l'étape c) ou d), le mélange est séparé par adsorption/désorption sur tamis moléculaires.

7. Procédé selon la revendication 6, caractérisé en ce qu'on utilise un tamis moléculaire minéral ayant une taille de pores de 5 à 6,5 A.

8. Procédé pour préparer des émulsions aqueuses de perfluorocarbones, pouvant assurer le transport de l'oxygène et utilisables en médecine, caractérisé en ce qu'on utilise comme constituant assurant le transfert de l'oxygène la perfluoro-N-cyclohexylmorpholine de formule I'a ou son mélange avec la perfluoro-N-n-hexylmorpholine de formule I'b
